# EUROPEAN PATENT APPLICATION

(11) **EP 3 505 062 A1**
(43) Date of publication of application: **03.07.2019**
(21) Application number: 17211182.5
(22) Date of filing: 29.12.2017
(51) Int. Cl.: A61B 5/103, A61B 5/145, A61B 5/00, A61B 5/026, A61H 23/00, A61H 7/00

(54) **A SKIN TREATMENT DEVICE**

(71) Applicant: Koninklijke Philips N.V., 5656 AE Eindhoven (NL)
(72) Inventor: JANSEN, Marjolein Yvonne, 5656 AE Eindhoven (NL); VAN DIJK, Tomas Rozinus, 5656 AE Eindhoven (NL); VAN AMERONGEN, Hendrik Halling, 5656 AE Eindhoven (NL); PEETERS, Felix Godfried Peter, 5656 AE Eindhoven (NL); YANG, Ke, 5656 AE Eindhoven (NL); KESSELS, Marijn, 5656 AE Eindhoven (NL)
(74) Representative: de Haan, Poul Erik

(57) **Abstract**

This invention relates to a skin treatment device (1) for treating a skin surface and a method for operating the skin treatment device (1). The skin treatment device (1) comprises a treatment unit (2) for performing skin treatment routines on the skin, a driving unit (3) for driving the treatment unit (2) and at least one sensor unit (4) configured to measure/sense information related to at least one of blood perfusion and color of the skin and determine a skin redness value by using the said information. The skin treatment device (1) further comprises a control unit (5) operably connected to the sensor unit (4) and configured to adjust the drive parameters of the driving unit (3) and/or provide a user notification based on the skin redness value.

## Description

### FIELD OF THE INVENTION

The present invention relates to a skin treatment device for treating a skin surface and method for operating the skin treatment device.

### BACKGROUND OF THE INVENTION

Blood circulation system brings nutrients such as oxygen, minerals, vitamins, and hormones to body parts and takes away toxins and wastes such as carbon dioxide, acid, protein byproducts. So skin benefits from an increase in blood perfusion. Having an increased blood perfusion throughout the skin helps for a healthy look and feel of the skin. However, to be perceived healthy, the redness of the skin should be balanced. Neither too red skin nor too pale skin would be desired for a healthy look.

There are various devices known to increase blood perfusion of the skin temporarily by performing skin treatment routines such as massaging, facial brushing, scrubbing, exfoliating. These devices generally increase skin redness by contacting the skin and stimulating the blood perfusion. However, if the level of skin redness increase is too high, the skin can reach a redness level that is not desired by the user. Moreover, blood perfusion sensitivity of the skin is different from person to person. Some people experience a large increase in skin redness after a light contact, while skin redness of others barely increases even after a complete workout.

WO2016046142 discloses a shaving device comprising a skin-contacting surface structure, a cutter, a shape-changing smart material and a controller for controlling activation of the shape-changing smart material during use of the shaving device. The shaving device further comprises a skin color sensor to determine the redness of the skin caused by skin irritation wherein the controller is configured to control said activation of the shape-changing smart material based on said skin color information received from the sensor.

### SUMMARY OF THE INVENTION

It is an object of the present invention to provide a skin treatment device with improved skin redness control.

In a first aspect of the invention, a skin treatment device (herein also indicated as "device") is presented. The device has a treatment unit which is driven by a driving unit. Driving unit is suitable to provide a rotational motion and/or vibratory motion. Driving unit is also suitable to provide light and/or radiofrequency energy. The device further comprises at least one sensor unit for measuring blood perfusion and/or the color of the skin. The sensor unit is configured to determine a skin redness value by using the sensor measurements. The skin redness value is used by a controlling unit for controlling the driving unit. Depending on the skin redness value, the control unit alters drive parameters of the driving unit and/or provides notification to the user. These drive parameters include the rotation speed and/or rotation direction of the rotational motion and/or the frequency and/or amplitude and/or an amplitude modulation of the vibratory motion and/or the operation duration. The drive parameters further include light intensity and/or light wavelength and/or RF energy level and/or RF energy frequency.

In an embodiment of the invention, the device comprises at least one warning means to provide notification to the user when the desired skin redness value is reached. The warning means is configured to provide audial and/or visual and/or haptic feedback to the user.

In an embodiment of the invention, the driving unit is controlled by using a predefined maximum skin redness value (Rₘ) stored in the memory of the control unit. During the treatment, the sensor unit makes real-time sensor measurements and determine the skin redness values accordingly. The control unit checks these skin redness values simultaneously. When the determined skin redness value reaches the predefined maximum skin redness value (Rₘ), the control unit adapts drive parameters such that the treatment process does not increase the skin redness value any more. This can be realized by either stopping the treatment or decreasing the speed/frequency of the driving unit.

In an alternative embodiment of the invention, the driving unit is controlled by using a predefined maximum skin redness increase value (ΔRₘ) stored in the memory of the control unit. In this embodiment, an initial skin redness value (Rᵢ) is determined at the beginning of the treatment and this value is subtracted from every single skin redness value determined afterwards to calculate a skin redness increase values (ΔR) during the treatment. The control unit checks these skin redness increase values (ΔR) simultaneously. When the skin redness increase value (ΔR) reaches the predefined maximum skin redness increase value (ΔRₘ), the control unit adapts drive parameters such that the treatment process does not increase the skin redness value any more. This can be realized by either stopping the treatment or decreasing the speed of the driving unit.

In another embodiment of the invention, when the predefined maximum skin redness value (Rₘ) or predefined maximum skin redness increase value (ΔRₘ) is reached, control unit provides user notification and/or slows down the treatment. Upon receiving the notification, user moves the device to an untreated part of the skin. In the meantime, sensor unit continues to determine the skin redness value. When the skin redness value drops below the predefined maximum skin redness value (Rₘ) or the predefined maximum skin redness increase value (ΔRₘ) again, the user notification is removed and/or treatment speed is adjusted back to the normal value until the predefined maximum skin redness value (Rₘ) or predefined maximum skin redness increase value (ΔRₘ) is reached again. This cycle is repeated until all relevant skin parts are treated.

In an embodiment of the invention, the predefined maximum skin redness value (Rₘ) or predefined maximum skin redness increase value (ΔRₘ) is adjustable by the user. This enables users to personalize the device in accordance with their skin characteristics.

In an embodiment of the invention, the device has two or more operating modes each of which has different predefined maximum skin redness values (Rₘ) or predefined maximum skin redness increase values (ΔRₘ). In a specific embodiment, the device has two modes, being a "light mode" and a "heavy mode" wherein the heavy mode has a higher predefined maximum skin redness value (Rₘ) or predefined maximum skin redness increase value (ΔRₘ) than the light mode. For example, the light mode is more suitable to be used before engaging in social contact in cases that the user would not like to have redness visible and the heavy mode is more suitable to be selected before sleeping to achieve a complete treatment without being concerned by the redness of the skin. This provides flexibility to use the device.

In an embodiment of the invention, the device has a configuration mode. In this embodiment, a test routine with predefined parameter settings is performed on the skin and several skin redness values are determined throughout the test routine. By using these skin redness values, a skin redness profile is created for every individual user. Personalized treatment can be configured by using this skin redness profile. Thus, sensitivity to redness factor, which is different for every person, can be eliminated.

In an embodiment of the invention, the sensor unit uses an optical sensor integrated into the device. In this embodiment, the optical sensor faces the skin during the treatment process. Thus, real-time measurements can be taken during the treatment. The sensor may be located either in the body or in the treatment unit of the device.

In a second aspect of the invention, a method for operating a skin treatment device is presented. The method comprises the steps of: determining an initial skin redness value; initiating the skin treatment routine; determining the skin redness value during the treatment; adjusting the drive parameters of the driving unit and/or providing user notification based on the change in the skin redness value.

In an embodiment of the invention, the step of "adjusting the drive parameters of the driving unit and/or providing user notification based on the change in the skin redness value" comprises sub-steps: comparing real time skin redness values determined during the treatment with the predefined maximum skin redness value and adjusting the drive parameters so as to stop or slow down the driving unit and/or provide user notification when the determined skin redness value is equal or higher than the predefined maximum skin redness value.

In an alternative embodiment of the invention, the step of "adjusting the drive parameters of the driving unit and/or providing user notification based on the change in the skin redness value" comprises sub-steps: calculating the skin redness increase value by subtracting the initial skin redness value from the real time skin redness value determined during the treatment; comparing the skin redness increase value with the predefined maximum skin redness increase value and adjusting the drive parameters so as to stop or slow down the driving unit and/or provide user notification when the calculated skin redness increase value is equal or higher than the predefined maximum skin redness increase value.

In an embodiment of the invention, the method further comprises: initiating a test routine with predefined parameters; determining a plurality of skin redness values during the test routine and generating a skin redness profile for an individual user based on the change of skin redness value throughout the test routine.

In a third aspect of the invention, there is provided a computer program comprising program code means for causing a computing device to carry out the steps of the methods described above when said computer program is carried out on a computing device.

According to the aspects and embodiments described above, it is possible to control the skin redness in a personalized way while the overall treatment efficiency of the skin treatment device is preserved.

These and other aspects will be apparent from and elucidated with reference to the embodiment(s) described hereinafter.

### BRIEF DESCRIPTION OF THE DRAWINGS

Embodiments of the invention will now be described, by way of example only, with reference to the accompanying drawings, in which:
Fig. 1 is a schematic perspective view of a skin treatment device in an embodiment;
Fig. 2 is a flow chart illustrating a method of operating a skin treatment device in an embodiment;
Fig. 3A is a flow chart illustrating sub-steps of the step (304) of the method illustrated in Fig. 2 in an embodiment of the invention;
Fig. 3B is a flow chart illustrating sub-steps of the step (304) of the method illustrated in Fig. 2 in another embodiment of the invention;
Fig. 4 is a flow chart illustrating further steps of the method illustrated in Fig. 2 in an embodiment of the invention.

The drawings are only schematic and are non-limiting. In the drawings, the size of some of the elements may be exaggerated and not drawn on scale for illustrative purposes.

### DETAILED DESCRIPTION OF EMBODIMENTS

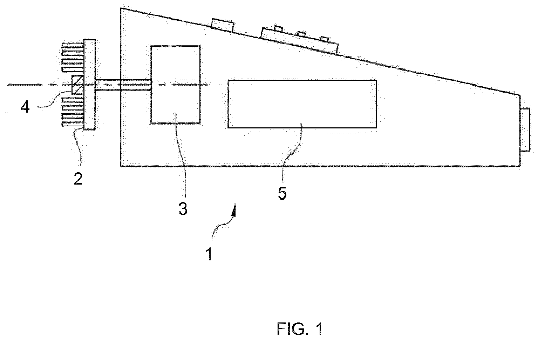

Fig. 1 illustrates a skin treatment device (1) for treating a skin surface according to an embodiment. As illustrated in Fig. 1, the device (1) comprises a treatment unit (2) configured to perform skin treatment routines on the skin and a driving unit (3) configured to drive the treatment unit (2). The driving unit (3) may drive the treatment unit (2) by applying rotational motion and/or axial vibratory motion. In some embodiments, either one of rotational motion or vibratory motion is used alone to drive the treatment unit (2). Alternatively, in some other embodiments, rotational motion and vibratory motion are used simultaneously. Although examples have been provided for motion type realized by the driving unit (3), it will be understood that any other type of motion can be used to drive the treatment unit (2). For example, driving unit (3) may provide light and/or radiofrequency (RF) energy to the treatment unit (2) wherein the device (1) is light or RF based skin treatment device.

As also illustrated in Fig. 1, the skin treatment device (1) further comprises at least one sensor unit (4) configured to measure/sense information related to at least one of blood perfusion and color of the skin and determine a skin redness value by using the said information. The sensor unit (4) may comprise any type of sensor for measuring blood perfusion and/or skin color. In some embodiments, the sensor unit (4) may comprise plurality of sensors and/or the device (1) may comprise a plurality of sensor units (4). Measurements taken from the sensor(s) are processed within the sensor unit (4) to determine a skin redness value. The skin redness value is a quantitative data calculated by the sensor unit (4) by using the measurements of the sensor(s) included in the sensor unit (4) and shows the redness level of the skin at the time of the measurement. The skin treatment device (1) further comprises a control unit (5). The control unit (5) is operably connected to the sensor unit (4) and configured to adjust the drive parameters of the driving unit (3) and/or provide user notification based on the skin redness value received from the sensor unit (4). The control unit (5) is configured to adjust drive parameters by stopping or slowing down the treatment when a desired skin redness value is reached. Alternatively, the control unit (5) provides notification to the user. To achieve this, a warning means is included in the device (1). Said warning means may be audial such as an alarming voice and/or visual such as a warning light on the body of the device (1). Alternatively, the warning means may be a haptic feedback such that the device (1) vibrates when a desired skin redness value is reached.

In an alternative embodiment the sensor unit (4) sends the raw measurement data to the control unit (5) and the control unit determines the skin redness value in accordance with the data provided.

The drive parameters include the rotation speed and/or rotation direction of the rotational motion and/or the frequency and/or amplitude and/or an amplitude modulation of the vibratory motion and/or the operation duration of the driving unit (3). The drive parameters further include light intensity and/or light wavelength and/or RF energy level and/or RF energy frequency.

In an embodiment of the invention, the sensor unit (4) is configured to determine the real time skin redness value during the treatment and the control unit (5) is configured to adjust the drive parameters so as to stop or slow down the driving unit (3) and/or provide user notification when the determined skin redness value reaches a predefined maximum skin redness value (Rₘ). In this embodiment, the sensor unit (4) continuously determines the skin redness value as long as the treatment unit (2) operates. The control unit (5) has at least one predefined maximum skin redness value (Rₘ) stored in its memory. Predefined maximum skin redness value (Rₘ) is selected so as to avoid a skin redness increase in a way discomforting the user. When the determined skin redness value is equal or higher than the predefined maximum skin redness value (Rₘ), control unit (5) adapts the drive parameters so as not to further increase the skin redness value. This is achieved either by decreasing the speed of the driving unit (3) or by stopping the driving unit (3). Alternatively, a user notification is provided by the warning means.

In an alternative embodiment of the invention, the sensor unit (4) is configured to determine an initial skin redness value (Rᵢ) at the beginning of the treatment and the control unit (5) is configured to calculate the skin redness increase value (ΔR) by subtracting the initial skin redness value (Ri) from the real time skin redness value determined during the treatment and adjusting the drive parameters so as to stop or slow down the driving unit (3) and/or providing user notification when the skin redness increase value (ΔR) reaches a predefined maximum skin redness increase value (ΔRₘ). The initial skin redness value (Rᵢ) can be determined at the beginning of the treatment or right before the treatment starts. After the initial skin redness value (Rᵢ) is determined and treatment starts, the sensor unit (4) continues to determine the skin redness value and simultaneously calculate a skin redness increase value (ΔR) for each determined skin redness value as long as the treatment unit (2) operates. The control unit (5) has at least one predefined maximum skin redness increase value (ΔRₘ) stored in its memory.

In another embodiment of the invention, when the predefined maximum skin redness value (Rₘ) or predefined maximum skin redness increase value (ΔRₘ) is reached, the control unit (5) provides a user notification and/or slows down the treatment. Upon receiving the notification, the user moves the device (1) to an untreated part of the skin. In the meantime, the sensor unit (4) keeps on determining skin redness values. When the skin redness value drops below the predefined maximum skin redness value (Rₘ) or the predefined maximum skin redness increase value (ΔRₘ) again, user notification is removed and/or the treatment speed is adjusted back to the normal value until the predefined maximum skin redness value (Rₘ) or predefined maximum skin redness increase value (ΔRₘ) is reached again. This cycle is repeated until the all relevant skin parts are treated.

Optionally, there may be an additional condition such as the completion percentage of the treatment, to decide stopping or slowing down the driving unit (3) when the predefined maximum skin redness value (Rₘ) or predefined maximum skin redness increase value (ΔRₘ) is reached. The completion percentage of the treatment may be calculated by dividing the elapsed treatment duration to the total treatment duration. In this embodiment, control unit (5) compares the completion percentage of the treatment at the time when the skin redness value reaches a predefined maximum skin redness value (Rₘ) or predefined maximum skin redness increase value (ΔRₘ), with a predefined completion percentage threshold, and stops the driving unit (3) if the said completion percentage is equal or higher than the predefined completion percentage threshold. If the said completion percentage is lower than the predefined completion percentage threshold, the control unit (5) decreases the speed of the driving unit (3) and continue to the treatment until the predefined completion percentage threshold is reached. In this way, the skin redness value can be controlled without harming the efficiency of the overall treatment.

In an embodiment of the invention, the predefined maximum skin redness value (Rₘ) or the predefined maximum skin redness increase value (ΔRₘ) are adjustable by the user. Thus, the user can set the ideal predefined maximum skin redness value (Rₘ) or the predefined maximum skin redness increase value (ΔRₘ) for himself/herself based on his/her previous experience and/or skin characteristics. This is realized either on the device (1) itself or via a mobile terminal such as mobile phone or tablet which is connectable to the device (1) and able to retrieve drive parameters and allows users to edit these parameters.

In an embodiment of the invention, treatment progress can be monitored from a mobile terminal such as mobile phone or tablet which is connectable to the device (1). In this embodiment, the determined skin redness values as well as other parameters related to the specific treatment can be simultaneously monitored from the mobile terminal during the treatment. Treatment data can be stored in a memory to further analyze the skin characteristic of the user and drive parameters used during the treatment.

In an embodiment of the invention, the device (1) has at least two operating modes with different predefined maximum skin redness values (Rₘ) or predefined maximum skin redness increase values (ΔRₘ). In a specific embodiment, the device (1) has two modes, namely a "light mode" and a "heavy mode" wherein the light mode has a lower predefined maximum skin redness value (Rₘ) or predefined maximum skin redness increase value (ΔRₘ) than the heavy mode. For example, the light mode is more suitable to be used before the user goes out for an activity where redness is not preferable and the heavy mode is more suitable to be selected before bedtime for a complete treatment when the user is not concerned by the redness of his/her skin. Each operation mode may have different drive parameters to increase the treatment efficiency of that specific mode. In other embodiments, the device (1) may have more than two operating modes.

In an embodiment of the invention, the device (1) has a configuration mode. In this mode, the treatment unit (2) is configured to perform a test routine with predefined parameter settings and the sensor unit (4) is configured to determine plurality of skin redness values during the test routine and generate a skin redness profile for an individual user based on the change of skin redness value throughout the test routine. The skin redness profile shows how sensitive the skin of the user is to the blood perfusion in terms of redness. In other terms, the skin redness profile also shows how quick the skin of the user turns red with a predefined treatment. During the test routine, some drive parameters are altered in various time intervals and the effect of those alterations on the skin redness value is determined. Thus, the skin redness profile also includes information about how sensitive the skin redness of the user to a change in a specific drive parameter. In this embodiment, the control unit (5) is configured to adjust the drive parameters in accordance with the skin redness profile of the individual user. Thus, a personalized treatment with optimized parameters in accordance with the skin redness profile can be performed.

In a version of this embodiment, the control unit (5) is configured to adjust the drive parameters such that the total operating duration remains unchanged. In this embodiment, after the initial skin redness value (Rᵢ) is determined, depending on the selected mode/control type, the skin redness value at which the treatment will be stopped is defined and the targeted skin redness increase value is calculated by the control unit (5). In an embodiment wherein the control unit (5) controls the driving unit (3) based on the predefined maximum skin redness increase value (ΔRₘ), the targeted skin redness increase value (ΔRₜ) is equal to the predefined maximum skin redness increase value (ΔRₘ) regardless of the initial skin redness value (Rᵢ). In an embodiment wherein the control unit (5) controls the driving unit (3) based on the predefined maximum skin redness value (Rₘ), the targeted skin redness increase value (ΔRₜ) is calculated by subtracting the initial skin redness value (Rᵢ) from the predefined maximum skin redness value (Rₘ). After the targeted skin redness increase value (ΔRₜ) is calculated, given that the total operating duration remains unchanged, a drive profile is generated by using varying drive parameters so as to achieve the targeted skin redness increase value (ΔRₜ) within the total operating duration. Thus, overall treatment efficiency is preserved while the skin redness is controlled.

In an embodiment of the invention, the sensor unit (4) includes an optical sensor integrated into the device (1). In this embodiment, the optical sensor is located so as to make measurements during the skin treatment operation of the device (1). For example, the device (1) may be a facial cleansing brush and the sensor may be located on a filament-free area of the brush unit such as the central part. Similarly, the device (1) may be a massaging device (1) and the sensor may be located on the massaging unit so as not contacting with but facing the skin. Sensor may be located either in the body or in the treatment unit (2) of the skin treatment device (1) in different embodiments.

In an embodiment of the invention, the device (1) is a facial skin treatment device such as a facial cleansing device, massaging device, scrubbing device, exfoliating device, microdermabrasion device, light based skin treatment device, radiofrequency (RF) based treatment device, etc.

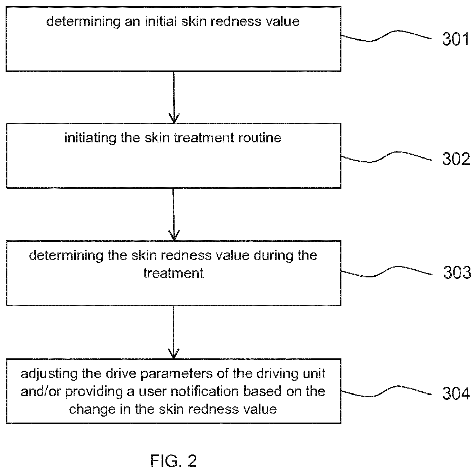

Fig. 2 is a flow chart illustrating a method of operating a skin treatment device. The method (300) comprises the steps of: determining an initial skin redness value (301); initiating the skin treatment routine (302); determining skin redness value during the treatment (303); adjusting the drive parameters of the driving unit and/or providing a user notification based on the change in the skin redness value (304).

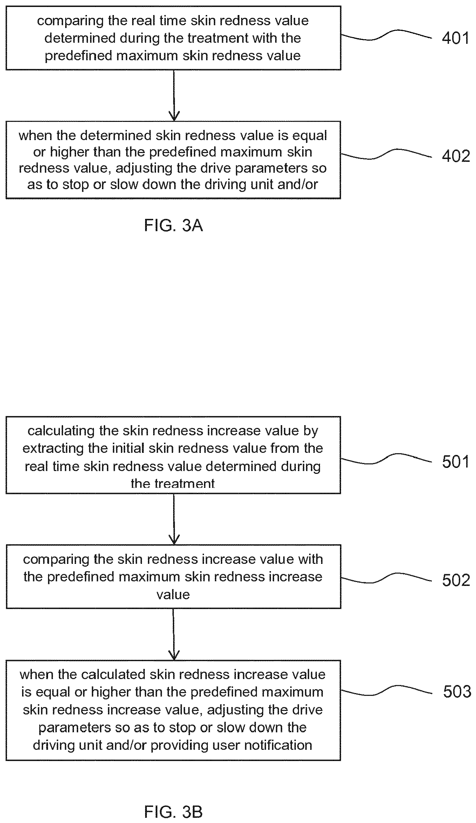

Fig. 3A is a flow chart illustrating sub-steps of step (304) in an embodiment of the invention. The method comprises: comparing real time skin redness values determined during the treatment with the predefined maximum skin redness value (401) and adjusting the drive parameters so as to stop or slow down the driving unit and/or providing a user notification when the determined skin redness value is equal or higher than the predefined maximum skin redness value (402).

Fig. 3B is a flow chart illustrating sub-steps of step (304) in an alternative embodiment of the invention. The method comprises: calculating the skin redness increase value by subtracting the initial skin redness value from the real time skin redness value determined during the treatment (501); comparing the skin redness increase value with the predefined maximum skin redness increase value (502) and adjusting the drive parameters so as to stop or slow down the driving unit and/or providing user notification when the calculated skin redness increase value is equal or higher than the predefined maximum skin redness increase value (503).

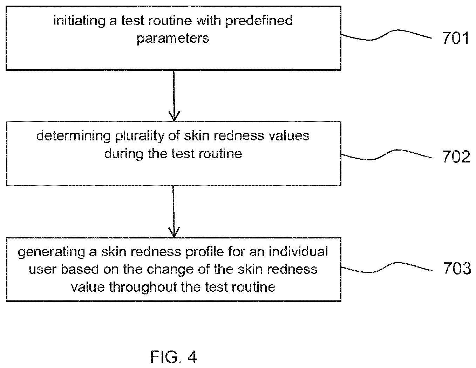

Fig. 4 is a flow chart illustrating further steps of the method (300) comprising: initiating a test routine with predefined parameters (701); determining a plurality of skin redness values during the test routine (702); generating a skin redness profile for an individual user based on the change of skin redness value throughout the test routine (703).

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A skin treatment device (1) for treating a skin surface, comprising:
a treatment unit (2) configured to perform skin treatment routines on the skin,
a driving unit (3) configured to drive the treatment unit (2)
at least one sensor unit (4) configured to measure/sense information related to at least one of blood perfusion and color of the skin and determine a skin redness value by using the said information,
a control unit (5) operably connected to the sensor unit (4) and configured to adjust the drive parameters of the driving unit (3) and/or provide a user notification based on the skin redness value.

2. A skin treatment device (1) as claimed in claim 1 wherein the sensor unit (4) is configured to determine the real time skin redness value during the treatment and the control unit (5) is configured to adjust the drive parameters so as to stop or slow down the driving unit (3) and/or provide user notification when the determined skin redness value reaches a predefined maximum skin redness value (Rₘ).

3. A skin treatment device (1) as claimed in claim 1 wherein the sensor unit (4) is configured to determine an initial skin redness value (Rᵢ) at the beginning of the treatment and the control unit (5) is configured to calculate the skin redness increase value (ΔR) by subtracting the initial skin redness value (Ri) from the real time skin redness value determined during the treatment and adjust the drive parameters so as to stop or slow down the driving unit (3) and/or provide a user notification when the skin redness increase value (ΔR) reaches a predefined maximum skin redness increase value (ΔRₘ).

4. A skin treatment device (1) as claimed in claim 2 or claim 3 wherein the predefined maximum skin redness value (Rₘ) and predefined maximum skin redness increase value (ΔRₘ) are adjustable by the user.

5. A skin treatment device (1) as claimed in any one of the preceding claims having at least two operating modes with different predefined maximum skin redness values (Rₘ) or predefined maximum skin redness increase values (ΔRₘ).

6. A skin treatment device (1) as claimed in any one of the preceding claims having a configuration mode wherein the treatment unit (2) is configured to perform a test routine with predefined parameter settings and wherein the sensor unit (4) is configured to determine a plurality of skin redness values during the test routine and generate a skin redness profile for an individual user based on the change of skin redness value throughout the test routine.

7. A skin treatment device (1) as claimed in claim 6 wherein the control unit (5) is configured to adjust drive parameters in accordance with the skin redness profile of the individual user.

8. A skin treatment device (1) as claimed in claim 7 wherein the control unit (5) is configured to adjust drive parameters such that the total operating duration remains unchanged.

9. A skin treatment device (1) as claimed in any one of the preceding claims, wherein the drive parameters include the rotation speed and/or rotation direction of the rotational motion and/or the frequency and/or amplitude and/or an amplitude modulation of the vibratory motion and/or the operation duration of the driving unit (3).

10. A skin treatment device (1) as claimed in any one of the preceding claims wherein the sensor unit (4) includes an optical sensor integrated into the device (1).

11. A method (300) of operating a skin treatment device, the method comprising the following steps:
determining an initial skin redness value (301),
initiating the skin treatment routine (302),
determining the skin redness value during the treatment (303),
adjusting the drive parameters of the driving unit and/or providing a user notification based on the change in the skin redness value (304).

12. A method (300) as claimed in claim 11 wherein the step (304) comprising the following sub-steps:
comparing the real time skin redness value determined during the treatment with the predefined maximum skin redness value (401)
when the determined skin redness value is equal or higher than the predefined maximum skin redness value, adjusting the drive parameters so as to stop or slow down the driving unit and/or providing user notification (402).

13. A method (300) as claimed in claim 11 wherein the step (304) comprising the following sub-steps:
calculating the skin redness increase value by subtracting the initial skin redness value from the real time skin redness value determined during the treatment (501)
comparing the skin redness increase value with the predefined maximum skin redness increase value (502)
when the calculated skin redness increase value is equal or higher than the predefined maximum skin redness increase value, adjusting the drive parameters so as to stop or slow down the driving unit and/or providing a user notification (503).

14. A method (300) as claimed in any one of claims 11 to 13 further comprising the steps of:
initiating a test routine with predefined parameters (701)
determining plurality of skin redness values during the test routine (702)
generating a skin redness profile for an individual user based on the change of the skin redness value throughout the test routine (703).

15. A computer program comprising program code means for causing a computing device to carry out the steps of the method as claimed in any one of claims 11 to 14 when said computer program is carried out on a computing device.
